# EUROPEAN PATENT SPECIFICATION

(11) **EP 3 993 796 B1**
(45) Date of publication and mention of the grant of the patent: **21.02.2024**
(21) Application number: 20739585.6
(22) Date of filing: 03.07.2020
(51) Int. Cl.: A61K 31/4985, A61K 31/65, A61P 13/00, A61P 15/00, A61P 31/04

(54) **COMBINATION FOR THE TREATMENT OF INFECTIONS CAUSED BY MYCOPLASMA GENITALIUM**
KOMBINATION ZUR BEHANDLUNG VON INFEKTIONEN DURCH MYCOPLASMA GENITALIUM
COMBINAISON POUR LE TRAITEMENT DES INFECTIONS CAUSÉES PAR MYCOPLASMA GENITALIUM

(30) Priority: 05.07.2019 US 201962870990 P; 05.03.2020 US 202062985465 P
(43) Date of publication of application: 11.05.2022
(73) Proprietor: GlaxoSmithKline Intellectual Property Development Limited, Stevenage SG1 2NY (GB)
(72) Inventor: SCANGARELLA-OMAN, Nicole, Collegeville, Pennsylvania 19426 (US)
(74) Representative: Jewson, Rie
(86) International application number: PCT/EP2020/068754
(87) International publication number: WO 2021/004910

(56) References cited:
- CATRIONA S BRADSHAW ET AL: "New Horizons in Mycoplasma genitalium Treatment", THE JOURNAL OF INFECTIOUS DISEASES, vol. 216, no. suppl_2, 15 July 2017 (2017-07-15), pages S412-S419, XP055731006, US ISSN: 0022-1899, DOI: 10.1093/infdis/jix132
- JENSEN JØRGEN SKOV: "Mycoplasma genitalium: yet another challenging STI", THE LANCET INFECTIOUS DISEASES, ELSEVIER, AMSTERDAM, NL, vol. 17, no. 8, 9 July 2017 (2017-07-09), pages 795-796, XP085137636, ISSN: 1473-3099, DOI: 10.1016/S1473-3099(17)30364-X
- JØRGEN SKOV JENSEN ET AL: "In vitro activity of the first-in-class triazaacenaphthylene gepotidacin alone and in combination with doxycycline against drug-resistant and -susceptible Mycoplasma genitalium", EMERGING MICROBES & INFECTIONS, vol. 9, no. 1, 18 June 2020 (2020-06-18), pages 1388-1392, XP055731026, DOI: 10.1080/22221751.2020.1775498
- JØRGEN JENSEN: "In vitro evaluation of gepotidacin, an oral antimicrobial against multidrug-resistant mycoplasma genitalium", SEXUALLY TRANSMITTED INFECTIONS, vol. 95, no. Supplement 1, P664, 14 July 2019 (2019-07-14), page A292, XP055731030, DOI: 10.1136/sextrans-2019-sti.731

## Description

This invention was made with Government support under The United States Of America Department Of Health And Human Services Assistant Secretary For Preparedness And Response, Biomedical Advanced Research and Development Authority (BARDA), within the Office of the Assistant Secretary for Preparedness and Response in the U.S. Department of Health and Human Services Agreement No.: HHSO100201300011C. The government has certain rights in this invention.

### FIELD OF THE INVENTION

The present invention relates to pharmaceutical combinations or compositions, combination or resistance guided therapies comprising gepotidacin or a pharmaceutically acceptable salt thereof and doxycycline for use in the treatment of a bacterial infection caused by *Mycoplasma genitalium.*

### BACKGROUND TO THE INVENTION

The misuse and overuse of antibiotics has led to concerning levels of global resistance rates across diseases, resulting in a world-wide call to action. Infections caused by multidrug-resistant organism(s) represent a major public health burden, not just in terms of morbidity and mortality, but also in terms of increased expenditure on patient management and implementation of infection control measures. The problem of antibacterial resistance is compounded by the existence of bacterial strains resistant to multiple antibacterials. Notably, antibiotic resistance in sexually transmitted infections (STIs) is a growing public health issue. STIs have the potential to become the first incurable bacterial infections since the introduction of antibiotics. *Mycoplasma genitalium* is an important cause of sexually transmitted infections (STIs) . *Mycoplasma genitalium* was first identified in the early 1980s and has become recognized as a cause of male urethritis, responsible for approximately 15%-20% of nongonococcal urethritis (NGU) cases, 20%-25% of nonchlamydial NGU, and approximately 30% of persistent or recurrent urethritis (Sexually Transmitted Diseases Treatment Guidelines 2015, Centers for Disease Control and Prevention). *Mycoplasma genitalium* has been detected in human urogenital, respiratory and rectal specimens but the human urogenital tract is considered to be its preferred site of colonization. As *Mycoplasma genitalium* is slow growing, detection is usually by a Nucleic Acid Amplification Test (NAAT).

*Mycoplasma genitalium* is associated with acute and chronic urethritis in men. Existing data on infection in women are limited and inconsistent but suggest that *Mycoplasma genitalium* is associated with urethritis, cervicitis, endometritis, pelvic inflammatory disease (PID), and possibly female infertility. *Mycoplasmagenitalium* lacks a cell wall, and thus common antibiotics targeting cell-wall biosynthesis (e.g., beta-lactams including penicillins and cephalosporins) are ineffective against this organism. Most *Mycoplasma genitalium* cases are asymptomatic and given diagnostic challenges, treatment of most infections caused by *Mycoplasma genitalium* will occur in the context of syndromic management for urethritis, cervicitis, endometritis, and PID.

Increasing reports of *Mycoplasma genitalium* resistance to traditional antibacterial agents such as macrolides and fluoroquinolones poses a challenge to successful treatment. In the "Sexually Transmitted Diseases Treatment Guidelines 2015, the Centers of Disease Control and Preventions, notes that azithromycin is the preferred first line treatment of an infection caused by *Mycoplasma genitalium,* but rates of resistance are increasing globally. Moxifloxacin is also used but the prevalence of mutations in the quinolone-resistance-determining-region (QRDR) of ParC is as high as 88% and dual resistance in have been reported. Another alternative, pristinamycin, is difficult to source and only 80% effective. With dual resistance to macrolides and fluoroquinolones, treatment options are extremely limited.

Bradshaw et al. (The Journal of Infectious Diseases, 2017:16, suppl_2, S412-419) and Jensen (The Lancet, 2017:17(8), 795-796) provide an overview of the antimicrobial therapies against *Mycoplasma genitalium* infections. Doxycycline is mentioned among other tetracyclines, and gepotidacin is mentioned as a new investigational agent.

Thus, there is a demand for development of novel pharmaceutical combinations or compositions, combination or resistance guided therapies and/or uses thereof containing a combination of antibiotic or antimicrobial agents with different mechanisms of action for treating bacterial infections, which demonstrate synergistic and bactericidal effects leading to less development of antibacterial or antimicrobial resistance. However, any combination of agents should not have an antagonistic interaction.

The present invention is directed to overcoming these and other problems encountered in the art.

### SUMMARY OF THE INVENTION

A novel-mechanism antibiotic, gepotidacin, has now been unexpectedly found to be synergistically effective against *Mycoplasma genitalium* when acting together with the tetracycline antibiotic, doxycycline, with no negative interaction.

The present invention provides doxycycline for use in the treatment of an infection caused by *Mycoplasma genitalium* by co-administration with gepotidacin or a pharmaceutically acceptable salt thereof.

The present invention also provides gepotidacin or a pharmaceutically acceptable salt thereof for use in the treatment of an infection caused by *Mycoplasma genitalium* by co-administration with doxycycline.

The present invention also provides a kit comprising gepotidacin or a pharmaceutically acceptable salt thereof, and doxycycline, for use in the treatment of an infection caused by *Mycoplasma genitalium.*

The present invention also provides a pharmaceutical combination which comprises gepotidacin or a pharmaceutically acceptable salt thereof and doxycycline for use in the treatment of an infection caused by *Mycoplasma genitalium.*

The present invention also provides a pharmaceutical composition which comprises gepotidacin or a pharmaceutically acceptable salt thereof, doxycycline, and at least one pharmaceutically acceptable excipient(s) for use in the treatment of an infection caused by *Mycoplasma genitalium.*

### DESCRIPTION OF DRAWINGS/FIGURES

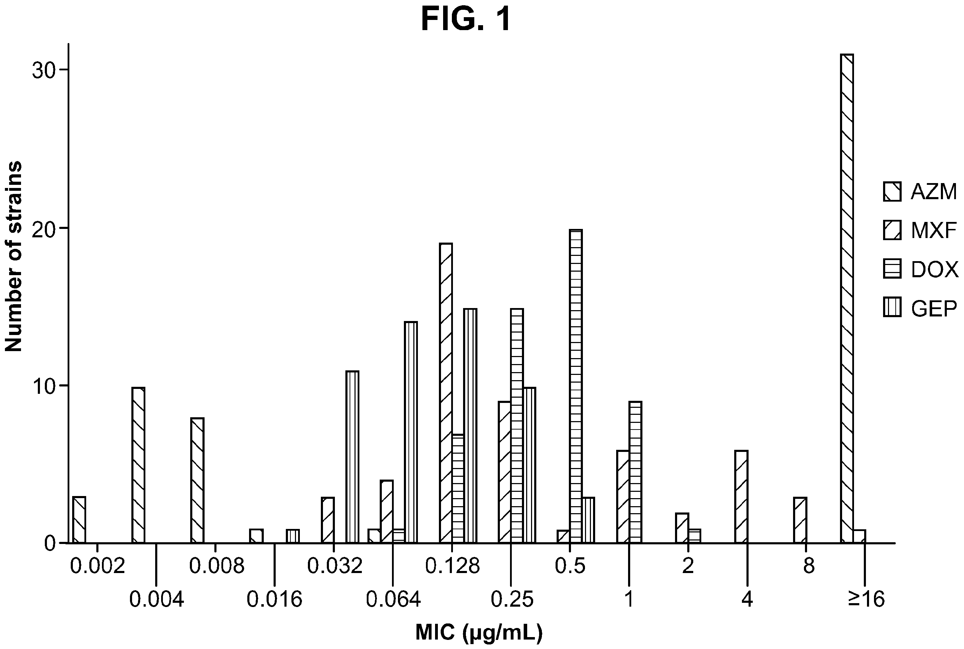

Figure 1 shows the distribution of Minimum Inhibitory Concentrations (MICs) in µg/mL for gepotidacin (GEP), azithromycin (AZM), moxifloxacin (MXF) and doxycycline (DOX), against 54 *Mycoplasma genitalium* strains as shown in Example 1.

### DETAILED DESCRIPTION OF THE INVENTION

The present invention is defined by the claims. Any subject-matter falling outside the scope of the claims is provided for information only. Methods of treatment of the human body by therapy, referred to in this description, are not part of the present invention as such, but are described here in relation to compounds and pharmaceutical compositions for use in said methods for treatment of the human body by therapy, according to the present invention. U

The terms "antimicrobial", "antibiotic" and "antibacterial" are used interchangeably in the present application, and refer to any natural or synthetic compound which kills or inhibits the growth of a microorganism.

Further as understood in the present invention, antibiotic resistance occurs when bacteria change in response to the use of antibiotics, making them ineffective; and antimicrobial resistance is a broader term, encompassing resistance to drugs that treat infections caused by other microbes as well, such as parasites (e.g. malaria or helminths), viruses (e.g. HIV) and fungi (e.g. *Candida).*

Doxycycline is a tetracycline antibiotic. It has been reported to have poor clinical efficacy in treatment of urogenital infections by *Mycoplasma genitalium* with cure rates of less than 50% (Manhart LE et al. Efficacy of antimicrobial therapy for infections by Mycoplasmagenitalium. Clin Infect Dis 2015; 61 (suppl 8): S802-17). As would be understood by the skilled person, the term "doxycycline" as used herein encompasses all forms of doxycycline including doxycycline hyclate (hydrochloride hemiethanolate hemihydrate) and doxycycline monohydrate.

Gepotidacin is a first-in-class, novel triazaacenaphthylene antibiotic with the ability to selectively inhibit bacterial DNA replication by a means not utilized by any currently approved human therapeutic agent, therefore providing the opportunity to address an unmet medical need. Gepotidacin and its racemic form is disclosed in WO 2088/128942.

Gepotidacin is (2*R*)-2-({4-[(3,4-dihydro-2*H*-pyrano[2,3-*c*]pyridin-6-ylmethyl)amino]-1-piperidinyl}methyl)-1,2-dihydro-3*H*,8*H-*2a,5,8a-triazaacenaphthylene-3,8-dione:

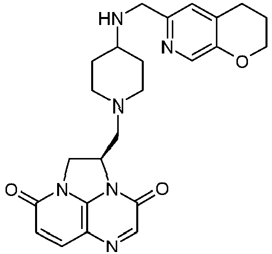

The treatments and combinations of the present invention are based on the combination of gepotidacin or a pharmaceutically acceptable salt thereof, and doxycycline. Combination therapy is a treatment in which a patient is given two or more drugs (or other therapeutic agents) for a single disease or when multiple diseases or pathogens are suspected or known to be present. Combination antibiotic therapy is used in patients due to widespread emergence of multidrug resistant (MDR) organisms. Multidrug resistance may be defined as lack of susceptibility to at least one agent in three or more antibiotic categories. Antimicrobials or antibacterials are frequently used in combination, so inhibitory drug interactions between the agents are undesirable.

Combination therapy may have the advantages of broadening antibacterial spectrum, providing synergistic effects, and discouraging the emergence of resistance.

The studies in the present application show a synergistic or additive effect when combining gepotidacin and doxycycline against *Mycoplasma genitalium.*

Thus, in the first aspect, the present invention provides combinations for use in the treatment of an infection caused by *Mycoplasma genitalium in* a human in need thereof, comprising a therapeutically effective amount of gepotidacin or a pharmaceutically acceptable salt thereof, with a therapeutically effective amount of doxycycline.

As used herein, "caused by *Mycoplasma genitalium"* may mean that *Mycoplasma genitalium* has been identified (e.g. by NAAT) as being the cause of an infection, or part of the cause of an infection (i.e. associated with the infection); or it may mean that *Mycoplasma genitalium* is suspected or strongly suspected to be the cause of the infection, or part of the cause of the infection, due to identification of symptoms and other factors such as patient history or local epidemiology.

In any of the aspects of the present invention, in one embodiment, the *Mycoplasma genitalium* causing the infection is resistant to a macrolide or a fluoroquinolone. Although it is recognised that "resistant" is a subset of "not-susceptible", as used herein, "resistant" is used synonymously with "not-susceptible" and refers to a form of the bacterium which resists the effects of, or has reduced or no susceptibility to, an antibiotic. In one embodiment, drug resistance of a *Mycoplasma genitalium* may be suspected (for example through knowledge of the local epidemiology or susceptibility patterns or patient's medical history, e.g. recurrent urethritis). In another embodiment, drug resistance may be proven through established techniques, which include phenotypic or genotypic determination. In one embodiment, in the present invention, "resistant" means resistance or not-susceptible as defined by *Performance Standards for Antimicrobial Susceptibility Testing,* Clinical and Laboratory Standards Institute (CLSI) supplement M100 and/or *Methods for Antimicrobial Susceptibility Testing for Human Mycoplasmas; Approved Guideline,* CLSI document M43-A: CLSI 2011. In one embodiment, the *Mycoplasma genitalium* causing the infection is resistant to azithromycin. In one embodiment, the *Mycoplasma genitalium* causing the infection is resistant to moxifloxacin.

In any of the aspects of the present invention, in one embodiment, the infection is selected from: sexually transmitted infections or diseases (STIs or STDs), bacterial causes of female infertility, genital tract infections and co-infection associated urinary tract infections. In one embodiment, the infection is urethritis. In one embodiment, the infection is acute urethritis, chronic urethritis, non-gonococcal urethritis or nonchlamydial non-gonococcal urethritis. In one embodiment, the infection is selected from urethritis, cervicitis, endometritis, and pelvic inflammatory disease (PID).

In one embodiment, the present invention provides a combination for use in the treatment of urethritis caused by *Mycoplasma genitalium* in a human in need thereof, comprising a therapeutically effective amount of gepotidacin or a pharmaceutically acceptable salt thereof, with a therapeutically effective amount of doxycycline.

In one embodiment, the present invention provides a combination for use in the treatment of acute urethritis caused by *Mycoplasma genitalium in* a human in need thereof, comprising a therapeutically effective amount of gepotidacin or a pharmaceutically acceptable salt thereof, with a therapeutically effective amount of doxycycline.

In one embodiment, the present invention provides a combination for use in the treatment of non-gonococcal urethritis caused by *Mycoplasma genitalium* in a human in need thereof, comprising a therapeutically effective amount of gepotidacin or a pharmaceutically acceptable salt thereof, with a therapeutically effective amount of doxycycline.

In any aspect of the present invention, the gepotidacin or a pharmaceutically acceptable salt thereof, and doxycycline, may be administered sequentially (i.e. serial administration), concurrently (i.e. co-administration) or simultaneously (i.e. simultaneous administration) in separate or combined pharmaceutical formulations by any convenient route.

In one embodiment, the gepotidacin or a pharmaceutically acceptable salt thereof, and doxycycline, are administered sequentially.

The administration of doxycycline may follow recommended treatment guidelines such as set out in "Sexually Transmitted Diseases Treatment Guidelines 2015", Centers for Disease Control and Prevention, or in "2016 European guideline on Mycoplasma genitalium Infections", Jensen et al, Journal of the European Academy of Dermatology and Venereology, DOI: 10.1111/jdv.13849. Thus for example, doxycycline may be given 200 mg orally once a day for 7 days, 100 mg orally twice a day for 7 days, or 100 mg orally twice a day for 14 days. Other possible treatment regimens include administering oral doxycycline 200 mg on the first day of treatment (as 100 mg every 12 hours) followed by a maintenance dose of 100 mg per day (which may be a single dose or 50 mg every 12 hours); or 100 mg every 12 hours.

Given the synergistic effect of doxycycline with gepotidacin as shown in the present application, the length of period of doxycycline administration may be reduced from 7 to 14 days to, for example, 1, 2, 3, 4, 5, 6, 7, 8, 9, 10, 11, 12 or 13 days.

In one embodiment, for any aspect of the present invention, doxycycline is administered for 5 days. In one embodiment, for any aspect of the present invention, doxycycline is administered for 6 days. In one embodiment, for any aspect of the present invention, doxycycline is administered for 7 days. In one embodiment, for any aspect of the present invention, doxycycline is administered for 8 days. In one embodiment, for any aspect of the present invention, doxycycline is administered for 9 days. In one embodiment, for any aspect of the present invention, doxycycline is administered for 10 days. In one embodiment, for any aspect of the present invention, doxycycline is administered for 11 days. In one embodiment, for any aspect of the present invention, doxycycline is administered for 12 days. In one embodiment, for any aspect of the present invention, doxycycline is administered for 13 days. In one embodiment, for any aspect of the present invention, doxycycline is administered for 14 days.

In one embodiment, for any aspect of the present invention, the human is administered gepotidacin or a pharmaceutically acceptable salt thereof for 1, 2, 3, 4, 5, 6, 7, 8, 9, 10, 11, 12, 13 or 14 days. In one embodiment, for any aspect of the present invention, the gepotidacin or a pharmaceutically acceptable salt thereof is administered at 1500 mg, b.i.d. (total daily dose 3000 mg) for 5 days. In another embodiment, for any aspect of the present invention, the gepotidacin or a pharmaceutically acceptable salt thereof is administered at 3000 mg once. In another embodiment, for any aspect of the present invention, the gepotidacin or a pharmaceutically acceptable salt thereof is administered at two doses of 3000 mg each, 6-12 hours apart.

In one embodiment, for any aspect of the present invention, the human is administered gepotidacin or a pharmaceutically acceptable salt thereof for 5 days. In one embodiment, for any aspect of the present invention, the human is administered gepotidacin or a pharmaceutically acceptable salt thereof for 6 days. In one embodiment, for any aspect of the present invention, the human is administered gepotidacin or a pharmaceutically acceptable salt thereof for 7 days. In one embodiment, for any aspect of the present invention, the human is administered gepotidacin or a pharmaceutically acceptable salt thereof for 8 days. In one embodiment, for any aspect of the present invention, the human is administered gepotidacin or a pharmaceutically acceptable salt thereof for 9 days. In one embodiment, for any aspect of the present invention, the human is administered gepotidacin or a pharmaceutically acceptable salt thereof for 10 days. In one embodiment, for any aspect of the present invention, the human is administered gepotidacin or a pharmaceutically acceptable salt thereof for 11 days. In one embodiment, for any aspect of the present invention, the human is administered gepotidacin or a pharmaceutically acceptable salt thereof for 12 days. In one embodiment, for any aspect of the present invention, the human is administered gepotidacin or a pharmaceutically acceptable salt thereof for 13 days. In one embodiment, for any aspect of the present invention, the human is administered gepotidacin or a pharmaceutically acceptable salt thereof for 14 days.

In another aspect, the present invention provides a combination for use in the treatment of an infection caused by *Mycoplasma genitalium* in a human in need thereof, comprising a therapeutically effective amount of doxycycline followed by a therapeutically effective amount of gepotidacin or a pharmaceutically acceptable salt thereof.

In one embodiment, the human is administered doxycycline for 1, 2, 3, 4, 5, 6, 7, 8, 9, 10, 11, 12, 13 or 14 days, followed by gepotidacin or a pharmaceutically acceptable salt thereof for 1, 2, 3, 4, 5, 6, 7, 8, 9, 10, 11, 12, 13 or 14 days. In one embodiment, the human is administered doxycycline for 7, 8, 9, 10, 11, 12, 13 or 14 days, followed by gepotidacin or a pharmaceutically acceptable salt thereof for 7, 8, 9, 10, 11, 12, 13 or 14 days.

In another aspect, the present invention provides a combination for use in the treatment of an infection caused by *Mycoplasma genitalium* in a human in need thereof, comprising a therapeutically effective amount of gepotidacin or a pharmaceutically acceptable salt thereof followed by a therapeutically effective amount of doxycycline.

In one embodiment, the human is administered gepotidacin or a pharmaceutically acceptable salt thereof for 1, 2, 3, 4, 5, 6, 7, 8, 9, 10, 11, 12, 13 or 14 days, followed by doxycycline for 1, 2, 3, 4, 5, 6, 7, 8, 9, 10, 11, 12, 13 or 14 days. In one embodiment, the human is administered gepotidacin or a pharmaceutically acceptable salt thereof for 7, 8, 9, 10, 11, 12, 13 or 14 days, followed by doxycycline for 7, 8, 9, 10, 11, 12, 13 or 14 days.

In one embodiment, for any aspect of the present invention, the total length of treatment is equal to or fewer than 14, 15, 16, 17, 18, 19, 20, 21, 22, 23, 24, 25, 26, 27 or 28 days.

In another aspect, the present invention provides a pharmaceutical composition for use in the treatment of an infection caused by *Mycoplasma genitalium* in a human in need thereof, said pharmaceutical composition comprising (a) a therapeutically effective amount of gepotidacin or a pharmaceutically acceptable salt thereof and (b) a therapeutically effective amount of doxycycline.

In one aspect, the present invention provides a combination for use in the treatment of an infection caused by *Mycoplasma genitalium* in a human in need thereof, comprising a therapeutically effective amount of gepotidacin or a pharmaceutically acceptable salt thereof, with a therapeutically effective amount of doxycycline, wherein the human has failed at least one prior line of therapy.

In one embodiment, the prior line of treatment may be an antibiotic such as a macrolide (e.g. azithromycin) or a fluoroquinolone (e.g. moxifloxacin). Failure of treatment may be defined according to established guidelines; for example, lack of improvement of symptoms after a 3, 4, 5, 6, or 7 day treatment with an antibiotic may be considered a failure.

In another aspect, the present invention provides a combination for use in the treatment of an infection caused by *Mycoplasma genitalium* in a human in need thereof, said treatment comprising the steps of:
a) determining whether the *Mycoplasma genitalium* strain causing the infection is resistant to macrolides or fluoroquinolones;
b) if the *Mycoplasma genitalium* strain is found to be resistant to macrolides or fluoroquinolones, administering to said human a therapeutically effective amount of gepotidacin or a pharmaceutically acceptable salt thereof, with a therapeutically effective amount of doxycycline.

Examples of macrolides include azithromycin, clarithromycin, erythromycin and josamycin. Examples of fluoroquinolones include ciprofloxacin, gemifloxacin, moxifloxacin and levofloxacin.

As used herein, "determining whether the *Mycoplasma genitalium* strain causing the infection is resistant to macrolides or fluoroquinolones" may mean that the skilled person considers local epidemiology, susceptibility patterns or patient history to determine likely resistance. Further, genetic and phenotypic methods known to the skilled person may be used.

In one embodiment, in step a), the *Mycoplasma genitalium* strain is tested for resistance to azithromycin. In another embodiment, in step a), the *Mycoplasma genitalium* strain is tested for resistance to moxifloxacin.

In one aspect, the present invention relates to a combination therapy for use in the treatment of a bacterial infection caused by *Mycoplasma genitalium,* comprising a therapeutically effective amount of gepotidacin or a pharmaceutically acceptable salt thereof, in combination with a therapeutically effective amount of doxycycline, to be administered to a human in need thereof.

In one embodiment, the infection is selected from: sexually transmitted infections or diseases (STIs or STDs), bacterial causes of female infertility, genital tract infections and co-infection associated urinary tract infections. In one embodiment, the infection is urethritis. In one embodiment, the infection is acute urethritis, chronic urethritis or non-gonococcal urethritis or nonchlamydial non-gonococcal urethritis. In one embodiment, the infection is selected from urethritis, cervicitis, endometritis, and pelvic inflammatory disease (PID).

In one embodiment, the present invention provides a combination therapy for use in the treatment of urethritis caused by *Mycoplasma genitalium,* comprising a therapeutically effective amount of gepotidacin or a pharmaceutically acceptable salt thereof, in combination with a therapeutically effective amount of doxycycline, to be administered to a human in need thereof.

In one embodiment, the present invention provides a combination therapy use in the treatment of non-gonococcal urethritis caused by *Mycoplasma genitalium,* comprising a therapeutically effective amount of gepotidacin or a pharmaceutically acceptable salt thereof, in combination with a therapeutically effective amount of doxycycline, to be administered to a human in need thereof.

The gepotidacin or a pharmaceutically acceptable salt thereof may be present in a pharmaceutical composition which comprises gepotidacin or a pharmaceutically acceptable salt thereof and at least one pharmaceutically acceptable excipient(s). Similarly, the doxycycline may be present in a corresponding pharmaceutical composition of doxycycline which comprises doxycycline and at least one pharmaceutically acceptable excipient(s).

In another aspect, the present invention relates to a combination or resistance guided therapy for use in the treatment of a bacterial infection caused by or associated with *Mycoplasma genitalium,* comprising a therapeutically effective amount of gepotidacin or a pharmaceutically acceptable salt thereof, in combination with a therapeutically effective amount of doxycycline, to be administered to a human in need thereof. The gepotidacin or a pharmaceutically acceptable salt thereof may be present in a pharmaceutical composition which comprises gepotidacin or a pharmaceutically acceptable salt thereof and at least one pharmaceutically acceptable excipient(s). Similarly, the doxycycline may be present in a corresponding pharmaceutical composition of doxycycline which comprises doxycycline and at least one pharmaceutically acceptable excipient(s). In one embodiment, the infection is urethritis. In one embodiment, the infection is acute urethritis, chronic urethritis or non-gonococcal urethritis.

As would be understood by the skilled person, as used herein, "resistance guided therapy" means a course of therapy, the direction of which is guided by knowledge of the phenotypic or genotypic susceptibility of the microorganism to a given antibiotic, for example as described in Bradshaw et al, The Journal of Infectious Diseases, Volume 216, Issue suppl_2, 15 July 2017, Pages S412-S419. Detecting *Mycoplasma genitalium* in an infection, then detecting the resistance of the *Mycoplasma genitalium* strain to certain antibiotics, in advance or during the course of treatment, has the advantage of potentially reducing the patient's exposure to ineffective antibiotics that may lead to resistance. Identification of *Mycoplasma genitalium* may be performed by any suitable means, such as by NAAT. Various assays capable of detecting macrolide resistance (either phenotypically or genetically) are also known.

Thus in one aspect, the present invention provides a resistance guided therapy for use in the treatment of a bacterial infection caused by *Mycoplasma genitalium,* comprising a therapeutically effective amount of gepotidacin or a pharmaceutically acceptable salt thereof, in combination with a therapeutically effective amount of doxycycline, to be administered to a human in need thereof.

The combination or resistance guided therapy of the present invention may be achieved by simultaneous administration, co-administration or serial administration of the two components.

In one aspect, the present invention relates to a combination or resistance guided therapy for use in the treatment of a bacterial infection caused by or associated with *Mycoplasmagenitalium,* where the bacterial infection is selected from: sexually transmitted infections or diseases (STIs or STDs), bacterial causes of female infertility, genital tract infections and co-infection associated urinary tract infections.

In one aspect, the present invention relates to a combination or resistance guided therapy for use in the treatment of a bacterial infection caused by or associated with *Mycoplasma genitalium,* where the genital tract infections or co-infection associated urinary tract infections, respectively, is selected from urethritis, cervicitis, endometritis, cervicitis, endometritis, endometritis or pelvic inflammatory disease (PID).

In one aspect, the present invention relates to a combination or resistance guided therapy for use in the treatment of a bacterial infection caused by or associated with *Mycoplasma genitalium,* where urethritis is selected from acute urethritis, chronic urethritis or non-gonococcal urethritis.

In one aspect, the present invention relates to a combination or resistance guided therapy for use in the treatment of a bacterial infection caused by or associated with *Mycoplasma genitalium,* where each of the components are administered orally.

In one aspect, the present invention relates to a combination or resistance guided therapy for use in the treatment of a bacterial infection caused by or associated with *Mycoplasma genitalium,* which comprises simultaneous administration, co-administration or serial administration of a therapeutically effective amount of gepotidacin or a pharmaceutically acceptable salt thereof and doxycycline, to a human in need thereof; where the combination with doxycycline results in a synergistic and/or additive effect; and/or also aids in protecting against development of resistance to either gepotidacin or a pharmaceutically acceptable salt thereof or doxycycline without interfering in its respective activity.

In one aspect, the present invention relates to a combination or resistance guided therapy for use in the treatment of a bacterial infection caused by or associated with *Mycoplasma genitalium,* which comprises simultaneous administration, co-administration or serial administration of a therapeutically effective amount of a pharmaceutical composition which comprises gepotidacin or a pharmaceutically acceptable salt thereof and at least one or more pharmaceutically acceptable excipient(s); and doxycycline, to a human in need thereof; where the combination with doxycycline results in a synergistic and/or additive effect; and/or also aids in protecting against development of resistance to either gepotidacin or a pharmaceutically acceptable salt thereof or doxycycline without interfering in its respective activity.

In one aspect, the present invention relates to a combination or resistance guided therapy for use in the treatment of genital tract infections or co-infection associated urinary tract infections caused by or associated with *Mycoplasma genitalium,* which comprises administration of therapeutically effective amount of gepotidacin or a pharmaceutically acceptable salt thereof; and doxycycline to a human in need thereof; where the combination with doxycycline results in a synergistic and/or additive effect; and/or also aids in protecting against development of resistance to either gepotidacin or a pharmaceutically acceptable salt thereof or doxycycline without interfering in their respective activities.

In one aspect, the present invention relates to a combination or resistance guided therapy for use in the treatment of urethritis caused by or associated with *Mycoplasma genitalium,* which comprises administration of therapeutically effective amount of gepotidacin; and doxycycline to a human in need thereof; where the combination with doxycycline results in a synergistic and/or additive effect; and/or also aids in protecting against development of resistance to either gepotidacin or a pharmaceutically acceptable salt thereof or doxycycline without interfering in their respective activities. In one embodiment, the urethritis is acute urethritis, chronic urethritis or non-gonococcal urethritis.

In one aspect, the present invention relates to a combination or resistance guided therapy for use in the treatment of non-gonococcal urethritis caused by or associated with *Mycoplasma genitalium,* which comprises administration of therapeutically effective amount of gepotidacin or a pharmaceutically acceptable salt thereof to a human in need thereof; and doxycycline to a human in need thereof, where use of doxycycline as the second antimicrobial or antibacterial agent results in a synergistic and/or additive effect; and/or also aids in protecting against development of resistance to either gepotidacin or a pharmaceutically acceptable salt thereof or doxycycline without interfering in its respective activity.

In one aspect, the present invention relates to a resistance guided therapy for use in the treatment of a bacterial infection caused by or associated with *Mycoplasma genitalium,* which comprises administration of a therapeutically effective amount of gepotidacin or a pharmaceutically acceptable salt thereof to a human in need thereof; and doxycycline, to a human in need thereof; where the combination with doxycycline results in a synergistic and/or additive effect; and/or also aids in protecting against development of resistance to either gepotidacin or a pharmaceutically acceptable salt thereof or doxycycline without interfering in their respective activity. In any of the above aspects and the embodiments of the present invention, in one embodiment, the human is male. In one embodiment, the human is female.

In another aspect, the present invention provides doxycycline for use in the treatment of an infection caused by *Mycoplasma genitalium* by co-administration with gepotidacin or a pharmaceutically acceptable salt thereof.

In another aspect, the present invention provides gepotidacin or a pharmaceutically acceptable salt thereof for use in the treatment of an infection caused by *Mycoplasma genitalium* by co-administration with doxycycline.

In one embodiment the infection is selected from: sexually transmitted infections (STIs), bacterial causes of female infertility, genital tract infections or co-infection associated urinary tract infections. In one embodiment, the genital tract infections or co-infection associated urinary tract infections is selected from urethritis, cervicitis, endometritis or pelvic inflammatory disease (PID). In one embodiment, the urethritis is selected from acute urethritis, chronic urethritis or non-gonococcal urethritis.

Thus in one embodiment, the present invention provides doxycycline for use in the treatment of urethritis caused by *Mycoplasma genitalium* by co-administration with gepotidacin or a pharmaceutically acceptable salt thereof.

In another aspect, the present invention provides gepotidacin or a pharmaceutically acceptable salt thereof for use in the treatment of urethritis caused by *Mycoplasma genitalium* by co-administration with doxycycline.

The gepotidacin or a pharmaceutically acceptable salt thereof, and doxycycline, may be administered sequentially or simultaneously in separate or combined pharmaceutical formulations by any convenient route.

In one embodiment the infection is selected from: sexually transmitted infections (STIs), bacterial causes of female infertility, genital tract infections or co-infection associated urinary tract infections. In one embodiment, the genital tract infections or co-infection associated urinary tract infections is selected from urethritis, cervicitis, endometritis or pelvic inflammatory disease (PID). In one embodiment, the urethritis is selected from acute urethritis, chronic urethritis or non-gonococcal urethritis.

In another aspect, the present invention provides a kit comprising gepotidacin or a pharmaceutically acceptable salt thereof, and doxycycline, for use in the treatment of an infection caused by *Mycoplasma genitalium.*

In another aspect, the present invention provides a pharmaceutical combination, which comprises gepotidacin or a pharmaceutically acceptable salt thereof; and doxycycline.

In another aspect, the present invention provides a pharmaceutical composition, which comprises gepotidacin or a pharmaceutically acceptable salt thereof, doxycycline, and at least one pharmaceutically acceptable excipient(s).

In another aspect, the present invention provides a pharmaceutical combination of gepotidacin or a pharmaceutically acceptable salt thereof, and doxycycline.

In another aspect, the present invention relates to a pharmaceutical combination, which comprises gepotidacin or a pharmaceutically acceptable salt thereof and doxycycline for use in combination or resistance guided therapy as described in the present invention.

In another aspect, the present invention relates to a pharmaceutical composition comprising gepotidacin or a pharmaceutically acceptable salt thereof, doxycycline, and at least one pharmaceutically acceptable excipient(s) for use in combination or resistance guided therapy as described in the present invention.

In another aspect, the present invention provides a combination of gepotidacin or a pharmaceutically acceptable salt thereof, and doxycycline, for use in the treatment of an infection caused by *Mycoplasma genitalium.*

In another aspect, the present invention provides a pharmaceutical composition comprising gepotidacin or a pharmaceutically acceptable salt thereof, and doxycycline, for use in the treatment of an infection caused by *Mycoplasma genitalium.*

### Compounds Used In The Present Invention

WO2008/128942 discloses the preparation of the free base and the hydrochloride salt of gepotidacin.

It will be understood that the phrase "gepotidacin or a pharmaceutically acceptable salt thereof" is intended to encompass gepotidacin, a pharmaceutically acceptable salt of gepotidacin, a solvate of gepotidacin, or any pharmaceutically acceptable combination of these. Thus by way of non-limiting example used here for illustrative purpose, "gepotidacin or a pharmaceutically acceptable salt thereof" may include a pharmaceutically acceptable salt of gepotidacin that is further present as a solvate.

As used herein, doxycycline or gepotidacin (or any pharmaceutically acceptable salt thereof) may be in any physical form thereof, including non-solid forms such as liquid or semi-solid forms, solid forms such as amorphous or crystalline forms, specific polymorphic forms and solvates including hydrates, for example doxycycline monohydrate and doxycycline hyclate (hydrochloride hemiethanolate hemihydrate), and mixtures of various forms.

Suitable pharmaceutically acceptable salts include those described by Berge, Bighley and Monkhouse J.Pharm.Sci (1977) 66, pp 1-19.

For both gepotidacin and doxycycline, a desired salt form may be prepared by any suitable method known in the art, including treatment of the free base with an inorganic acid, such as hydrochloric acid, hydrobromic acid, sulfuric acid, nitric acid, phosphoric acid, and the like, or with an organic acid, such as acetic acid, trifluoroacetic acid, maleic acid, succinic acid, mandelic acid, fumaric acid, malonic acid, pyruvic acid, oxalic acid, glycolic acid, salicylic acid, pyranosidyl acid, such as glucuronic acid or galacturonic acid, alpha-hydroxy acid, such as citric acid or tartaric acid, amino acid, such as aspartic acid or glutamic acid, aromatic acid, such as benzoic acid or cinnamic acid, sulfonic acid, such as p-toluenesulfonic acid, methanesulfonic acid, ethanesulfonic acid or the like. Examples of pharmaceutically acceptable salts include sulfates, pyrosulfates, bisulfates, sulfites, bisulfites, phosphates, chlorides, bromides, iodides, acetates, propionates, decanoates, caprylates, acrylates, formates, isobutyrates, caproates, heptanoates, propiolates, oxalates, malonates succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, phenylacetates, phenylpropionates, phenylbutrates, citrates, lactates, γ-hydroxybutyrates, glycollates, tartrates mandelates, and sulfonates, such as xylenesulfonates, methanesulfonates, propanesulfonates, naphthalene-1-sulfonates and naphthalene-2-sulfonates.

Pharmaceutically acceptable salts of gepotidacin include the acid addition salts, for example their salts with mineral acids e.g. hydrochloric, hydrobromic, sulphuric nitric or phosphoric acids, or organic acids, e.g. acetic, fumaric, succinic, maleic, citric, benzoic, p-toluenesulphonic, methanesulphonic, naphthalenesulphonic acid or tartaric acids. In one embodiment, in any aspect of the invention, the gepotidacin is gepotidacin free base or is gepotidacin methanesulphonate (mesylate).

The present invention includes within its scope all possible stoichiometric and non-stoichiometric salt forms.

### Pharmaceutical Compositions And Formulations

Pharmaceutical compositions and formulations acceptable and adaptable for use in methods and/or uses of the present invention are prepared using conventional art known pharmaceutical compositions, formulation or chemical materials, formulary excipients, preparation means, processes and/or methods and conventional techniques, etc.

In particular, gepotidacin or pharmaceutically acceptable salts, used in the present invention may be formulated for administration in any convenient way for use in human or veterinary medicine, by analogy with other antibacterials/antitubercular compounds.

The pharmaceutical compositions used in the present invention may be formulated for administration by any route and include those in a form adapted for oral, topical or parenteral use and may be used in mammals including humans.

The compositions may be in the form of tablets, capsules, powders, granules, lozenges, suppositories, creams or liquid preparations, such as oral or sterile parenteral solutions or suspensions.

In one embodiment, the gepotidacin or pharmaceutically acceptable salt thereof of the present invention is in a tablet or a capsule form. In one embodiment, it is in a tablet form. In one embodiment, the tablet is a 750mg tablet.

The doxycycline may be administered in any suitable form, including oral capsule, oral tablet, delayed or extended release oral tablet, delayed or extended release oral capsule, oral suspension (i.e. dry powder for reconstitution with water) or injectable solution.

Tablets and capsules for oral administration in the present invention may be in unit dose presentation form, and may contain conventional excipients such as binding agents, fillers, tabletting lubricants, disintegrants, or wetting agents. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

Moreover, the quantity of the compound or pharmaceutical composition used in the present invention administered will vary depending on the patient and the mode of administration and can be any effective amount.

In accordance with any of the methods of administration of the present invention, the term a "therapeutically effective amount", as used herein, generally includes within its meaning a non-toxic but sufficient amount of the particular drug to which it is referring to provide the desired therapeutic effect. The exact amount required will vary from subject to subject depending on factors such as the patient's general health, the patient's age, etc.

Treatment regimens for the administration of the compounds and/or pharmaceutical compositions used in the present invention can also be determined readily by those with ordinary skill in art. The quantity of the compound and/or pharmaceutical composition used in the present invention administered may vary over a wide range to provide in a unit dosage an effective amount based upon the body weight of the patient per day to achieve the desired effect.

The compositions may contain from 0.1% by weight, preferably from 10-60% by weight, of the active material, depending on the method of administration. Where the compositions comprise dosage units, each unit will preferably contain from 50-1000 mg of the active ingredient. Unless otherwise noted, the amount of the active ingredient (i.e., gepotidacin) refers to that of gepotidacin free base.

The dosage of the gepotidacin or a pharmaceutically acceptable salt as employed for adult human treatment in the present invention will preferably range from 100 or 6000 mg per day, or 100 to 3000 mg per day, for instance 1500 mg per day or 3000 mg per day depending on the route and frequency of administration. Such a dosage corresponds to about 1.5 to about 100 or 50 mg/kg per day. Suitably the dosage is from 5 to 30 mg/kg per day. In one embodiment, the dosage is 1500 mg twice a day (i.e. 3000 mg per day). In one embodiment, the dosage is 3000 mg twice a day (i.e. 6000 mg per day).

Conventional administration methods may be suitable for use in the present invention.

Depending upon the treatment being effected, the compounds, and/or or compositions of the present invention can be administered orally, intravascularly, intraperitoneally, subcutaneously, intramuscularly or topically. Preferably, the composition is adapted for oral administration. In any of the above aspects of the present invention, in one embodiment, the gepotidacin or a pharmaceutically acceptable salt thereof and the doxycycline are administered orally.

The Examples set forth below are illustrative of the present invention and are not intended to limit, in any way, the scope of the present invention.

### EXAMPLES

### EXAMPLE 1: In Vitro Evaluation Of Gepotidacin, An Oral Antimicrobial Against Drug-Resistant Mycoplasma Genitalium

### Summary

Gepotidacin minimal inhibitory concentrations (MICs) and minimal bactericidal concentrations (MBCs) were determined against 54 *Mycoplasma genitalium* isolates by the Vero-cell culture method. Macrolide resistance was present in 31 isolates, fluoroquinolone resistance in 18 (33%) isolates; 17 had dual resistance.

Synergy testing was performed for gepotidacin and doxycycline by checkerboard analysis for two macrolide-resistant and two dual-resistant isolates.

Gepotidacin was active against all 54 *Mycoplasma genitalium* isolates with median and modal MICs of 0.125 mg/L and a MIC₉₀ at 0.25 mg/L (range ≤0.016-0.5 mg/L). No difference in gepotidacin MIC between macrolide-resistant and -susceptible isolates (p=0.24) or between fluoroquinolone-. dual-resistant and -susceptible isolates (p=0.2) was demonstrated.

Gepotidacin MBCs were available for 44 *Mycoplasma genitalium* isolates with a median MIC of 0.064 and a median MBC of 0.125 mg/L and all isolates had ≤4-fold difference between MIC and MBC suggesting a bactericidal effect.

Checkerboard analysis indicated a synergistic effect for gepotidacin in combination with doxycycline with a Fractional Inhibitory Concentration Index (FIC) of 0.5 for two isolates (one macrolide-resistant and one dual-resistant) and additive/indifference (FIC at 0.62 and 0.75) for a macrolide- and a dual-resistant isolate, respectively.

### Methods

### Mycoplasma genitalium isolates

A collection of 54 *Mycoplasma genitalium* isolates originating from 51 patients were tested. Thirty-one strains were macrolide-resistant, with azithromycin MICs of ≥16 µg/mL and mutations in 23S rRNA gene positions A2058 (n=17) or A2059 (n=14, *E. coli* numbering). Eighteen strains were moxifloxacin-resistant (MIC ≥1 pg/ml); 17 had dual resistance and were resistant to both moxifloxacin and azithromycin. The geographical origins of the isolates are shown in Table 1.

**Table 1: Distribution Of 54 Mycoplasma genitalium Isolates According To Country Of Origin And Antimicrobial Resistance.**

| Country Of Origin | Number Of Isolates | Number Of Macrolide-resistant isolates | Number of Fluoroquinolone-resistant Isolates | Number Of Dual-class resistant Isolates |
|---|---|---|---|---|
| UK | 3 | 1 | 1 | 1 |
| Denmark | 9 | 4 | 3 | 3 |
| Sweden | 15 | 6 | 2 | 2 |
| Norway | 7 | 7 | 5 | 5 |
| France | 3 | 0 | 0 | 0 |
| Japan | 3 | 0 | 1 | 0 |
| Australia | 13 | 12 | 6 | 6 |
| USA | 1 | 1 | 0 | 0 |

### Determination Of Minimum Inhibitory Concentration (MIC)

Gepotidacin minimal inhibitory concentrations (MICs) were determined by the Vero-cell culture method for all 54 isolates and minimal bactericidal concentrations (MBCs) against 44 *Mycoplasma genitalium* strains. Gepotidacin, azithromycin, doxycycline, and moxifloxacin MICs were determined by inoculating 5000 genome equivalents (geq) as determined by quantitative PCR (qPCR) into a Vero-cell culture containing two-fold dilutions of test-antibiotic (Hamasuna et al., Antimicrob. Agents Chemother 49, 4993-4998 (2005)). After a three-week incubation period, cells and supernatant were harvested and growth of *Mycoplasma genitalium* was determined by qPCR. MIC was defined as the minimal concentration of the test-antibiotic causing a 99% inhibition of growth when compared to the mean of the control cultures grown without antibiotic.

### Determination Of Minimum Bactericidal Concentration (MBC)

After incubation for MIC determination, 15 µl of culture medium was transferred from the MIC plate to 135 µl fresh Vero-cell suspension, resulting in a 10-fold dilution of gepotidacin. Plates were sealed and incubated for four weeks and *Mycoplasma genitalium* growth was subsequently determined by qPCR. MBC was defined as the minimal concentration of the test-antibiotic causing a 99% inhibition of growth when compared to the mean of the control culture wells grown without antibiotic.

### Determination Of Synergy Between Gepotidacin And Doxycycline

Synergy testing was performed for gepotidacin and doxycycline by checkerboard analysis for two macrolide-resistant and two dual-resistant isolates. Checkerboards representing 8 by 8 two-fold dilutions of gepotidacin and doxycycline, with the mid-point concentration representing the doxycycline and gepotidacin MICs, respectively was prepared in Vero cell suspensions and *Mycoplasma genitalium* was incubated for three weeks before growth was determined by qPCR. Results were expressed as the fractional inhibitory concentration index (FICI). Synergy was assumed when FICI ≤ 0.5, antagonism when FICI >4.

### Results and Discussion

Gepotidacin was active against all 54 *Mycoplasma genitalium* isolates with a median and a modal MIC of 0.125 mg/L and a MIC₉₀ of 0.25 mg/L (range ≤0.016-0.5 mg/L) and was significantly more active than the comparator antibiotics against the *Mycoplasma genitalium* isolates tested (see Figure 1, which shows the distribution of MICs (ug/mL) for gepotidacin (GEP), azithromycin (AZM), moxifloxacin (MXF) and doxycycline (DOX) against 54 *Mycoplasma genitalium* strains). These included geographically, temporally and genetically diverse reference strains and clinical *Mycoplasma genitalium* isolates with a high proportion of fluoroquinolone- (33%) and macrolide-resistant (57%) isolates.

Importantly, no difference in gepotidacin MIC between macrolide-resistant and -susceptible isolates (p=0.24) or between fluoroquinolone-, dual-resistant or -susceptible isolates (p=0.2) was demonstrated.

In this selected collection of isolates, gepotidacin was the most active compound (Table 2) with an MIC₉₀ of 0.25 µg/ml compared with azithromycin (MIC₉₀ of >64 µg/ml), moxifloxacin (MIC₉₀ of 4 µg/ml), and doxycycline (MIC₉₀ of 1 pg/ml) (p<0.001 for all comparisons).

**Table 2: MICs of 36 moxifloxacin-susceptible and 18-moxifloxacin resistant isolates of Mycoplasma genitalium**

| Gepotidacin MBCs were available for 44 *Mycoplasma genitalium* isolates with a median MIC of 0.064 and a median MBC of 0.125 mg/L and all isolates had ≤4 fold difference between MIC and MBC suggesting bactericidal effect. | | | |
|---|---|---|---|
| Antibiotic | MIC₅₀ [µg/ml] | MIC₉₀ [µg/ml] | MIC range [µg/ml] |
| Gepotidacin | 0.125 | 0.25 | ≤0.016-0.5 |
| Gepotidacin (moxifloxacin-susceptible) | 0.125 | 0.25 | 0.032-0.5 |
| Gepotidacin (moxifloxacin-resistant) | 0.064 | 0.25 | ≤0.016-0.5 |
| Azithromycin | 16 | >64 | 0.002->64 |
| Doxycycline | 0.5 | 1 | 0.06-2 |
| Moxifloxacin | 0.25 | 4 | 0.03-> 16 |

Checkerboard analysis of gepotidacin and doxycycline indicated a synergistic effect with a Fractional Inhibitory Concentration Index (FIC) of 0.5 for two isolates (one macrolide-resistant and one dual-resistant) and an additive/indifferent effect (FIC at 0.62 and 0.75) for a macrolide- and a dual-resistant isolate, respectively (Table 3). Importantly, no antagonistic interactions between gepotidacin and doxycycline were identified.

For the purpose of this study and in Table 3, synergy was defined as FICI ≤0.5 and antagonism as FICI>4,macrolide resistance mediating mutations in the 23S rRNA gene were according to *E. coli* numbering and ParC mutations leading to moxifloxacin resistance were according to *Mycoplasma genitalium* numbering.

**Table 3. Fractional inhibitory concentration index (FICI) and MIC for selected antimicrobials in mg/L for four Mycoplasma genitalium isolates**

| | FICI DOX/GE P | 23S rRNA gene mutatio n | ParC mutatio n | GyrA mutatio n | Doxycycli ne | Azithromyc in | Moxifloxac in | Gepotidac in |
|---|---|---|---|---|---|---|---|---|
| M625 7 | 0.5 | A2058 G | WT | WT | 0.5 | >64 | 0.25 | 0.032 |
| M632 0 | 0.62 | A2059 G | WT | WT | 0.25 | ≥64 | 0.063 | 0.125 |
| M692 6 | 0.75 | A2058T | S83I | WT | 0.5 | 16 | 4 | 0.064 |
| M698 4 | 0.5 | A2058 G | D87N | WT | 0.5 | >64 | 1 | 0.125 |

These results show that gepotidacin was active against all *Mycoplasma genitalium* isolates tested, including fluoroquinolone- and macrolide-resistant isolates with no cross-resistance to the tested comparators. Gepotidacin MIC values were higher using the Vero-cell culture method than that found using CLSI methods for testing of moxifloxacin-susceptible *Mycoplasma genitalium* isolates, but bactericidal concentrations were comparable. Furthermore, the results show that doxycycline had an additive or synergistic effect with gepotidacin, even in *Mycoplasma genitalium* isolates with dual resistance.

The synergistic or additive effect may help to shorten the patient's period of exposure to the first drug (which may be doxycycline or gepotidacin) to a few days, rather than a lengthy period, before switching to the other drug, or adding the other drug to the treatment regimen. This dual therapy may help protect gepotidacin from selection and/or spread of resistance as well as increase the potency of both compounds.

It is to be understood that the invention is not limited to the aspects or embodiments illustrated hereinabove and the right is reserved to the illustrated aspects or embodiments falling within the scope of the following claims.

## Claims

1. Doxycycline for use in the treatment of an infection caused by *Mycoplasma genitalium* by co-administration with gepotidacin or a pharmaceutically acceptable salt thereof.

2. Gepotidacin or a pharmaceutically acceptable salt thereof for use in the treatment of an infection caused by *Mycoplasma genitalium* by co-administration with doxycycline.

3. Doxycycline or gepotidacin or a pharmaceutically acceptable salt thereof for use as claimed in claim 1 or claim 2, wherein the infection is selected from: sexually transmitted infections or diseases, bacterial causes of female infertility, genital tract infections and co- infection associated urinary tract infections.

4. Doxycycline or gepotidacin or a pharmaceutically acceptable salt thereof for use as claimed in claim 1 or claim 2, wherein the infection is urethritis.

5. Doxycycline or gepotidacin or a pharmaceutically acceptable salt thereof for use as claimed in claim 4, wherein the infection is non-gonococcal urethritis.

6. Doxycycline or gepotidacin or a pharmaceutically acceptable salt thereof for use as claimed in any of claims 1-5, wherein the *Mycoplasma genitalium* causing the infection is resistant to azithromycin or moxifloxacin.

7. Doxycycline or gepotidacin or a pharmaceutically acceptable salt thereof for use as claimed in any of claims 1-6, wherein the gepotidacin or a pharmaceutically acceptable salt thereof, and doxycycline, are administered sequentially.

8. Gepotidacin or a pharmaceutically acceptable salt thereof for use as claimed in any of claims 1-7, wherein the gepotidacin is gepotidacin methanesulphonate.

9. Gepotidacin or a pharmaceutically acceptable salt thereof for use as claimed in any of claims 1-8, wherein the gepotidacin or a pharmaceutically acceptable salt thereof is administered orally.

10. Doxycycline for use as claimed in any of claims 1-7, wherein the doxycycline is administered orally.

11. A kit comprising gepotidacin or a pharmaceutically acceptable salt thereof, and doxycycline, for use in the treatment of an infection caused by *Mycoplasma genitalium.*

12. A pharmaceutical combination which comprises gepotidacin or a pharmaceutically acceptable salt thereof and doxycycline for use in the treatment of an infection caused by *Mycoplasma genitalium.*

13. A pharmaceutical composition which comprises gepotidacin or a pharmaceutically acceptable salt thereof, doxycycline, and at least one pharmaceutically acceptable excipient(s) for use in the treatment of an infection caused by *Mycoplasma genitalium.*

## Patentansprüche

1. Doxycyclin zur Verwendung bei der Behandlung einer durch *Mycoplasma genitalium* verursachten Infektion durch gleichzeitige Verabreichung mit Gepotidacin oder einem pharmazeutisch akzeptablen Salz davon.

2. Gepotidacin oder ein pharmazeutisch akzeptables Salz davon zur Verwendung bei der Behandlung einer durch *Mycoplasma genitalium* verursachten Infektion durch gleichzeitige Verabreichung mit Doxycyclin.

3. Doxycyclin oder Gepotidacin oder ein pharmazeutisch akzeptables Salz davon zur Verwendung gemäß Anspruch 1 oder Anspruch 2, wobei die Infektion ausgewählt ist aus: sexuell übertragbaren Infektionen oder Erkrankungen, bakteriellen Ursachen weiblicher Unfruchtbarkeit, Infektionen des Genitaltrakts und co-infektionsbedingten Harnwegsinfektionen.

4. Doxycyclin oder Gepotidacin oder ein pharmazeutisch akzeptables Salz davon zur Verwendung gemäß Anspruch 1 oder Anspruch 2, wobei die Infektion Urethritis ist.

5. Doxycyclin oder Gepotidacin oder ein pharmazeutisch akzeptables Salz davon zur Verwendung gemäß Anspruch 4, wobei die Infektion nichtgonorrhoische Urethritis ist.

6. Doxycyclin oder Gepotidacin oder ein pharmazeutisch akzeptables Salz davon zur Verwendung gemäß irgendeinem der Ansprüche 1-5, wobei das die Infektion verursachende *Mycoplasma genitalium* resistent gegen Azithromycin oder Moxifloxacin ist.

7. Doxycyclin oder Gepotidacin oder ein pharmazeutisch akzeptables Salz davon zur Verwendung gemäß irgendeinem der Ansprüche 1-6, wobei das Gepotidacin oder ein pharmazeutisch akzeptables Salz davon und Doxycyclin sequentiell verabreicht werden.

8. Gepotidacin oder ein pharmazeutisch akzeptables Salz davon zur Verwendung gemäß irgendeinem der Ansprüche 1-7, wobei das Gepotidacin Gepotidacin-Methansulfonat ist.

9. Gepotidacin oder ein pharmazeutisch akzeptables Salz davon zur Verwendung gemäß irgendeinem der Ansprüche 1-8, wobei das Gepotidacin oder ein pharmazeutisch akzeptables Salz davon oral verabreicht wird.

10. Doxycyclin zur Verwendung gemäß irgendeinem der Ansprüche 1-7, wobei das Doxycyclin oral verabreicht wird.

11. Kit, umfassend Gepotidacin oder ein pharmazeutisch akzeptables Salz davon und Doxycyclin, zur Verwendung bei der Behandlung einer durch *Mycoplasma genitalium* verursachten Infektion.

12. Pharmazeutische Kombination, umfassend Gepotidacin oder ein pharmazeutisch akzeptables Salz davon und Doxycyclin, zur Verwendung bei der Behandlung einer durch *Mycoplasma genitalium* verursachten Infektion.

13. Pharmazeutische Zusammensetzung, umfassend Gepotidacin oder ein pharmazeutisch akzeptables Salz davon, Doxycyclin und wenigstens einen pharmazeutisch akzeptable(n) Hilfsstoff(e), zur Verwendung bei der Behandlung einer durch *Mycoplasma genitalium* verursachten Infektion.

## Revendications

1. Doxycycline destinée à être utilisée dans le traitement d'une infection causée par *Mycoplasma genitalium* par co-administration avec la gépotidacine ou un sel pharmaceutiquement acceptable de celle-ci.

2. Gépotidacine ou un sel pharmaceutiquement acceptable de celle-ci destinée à être utilisée dans le traitement d'une infection causée par *Mycoplasma genitalium* par co-administration avec la doxycycline.

3. Doxycycline ou gépotidacine ou un sel pharmaceutiquement acceptable de celle-ci destinée à être utilisée selon la revendication 1 ou la revendication 2, où l'infection est choisie parmi: les infections ou maladies transmises sexuellement, les causes bactériennes de l'infertilité féminine, les infections des voies génitales et les infections des voies urinaires associées à une co-infection.

4. Doxycycline ou gépotidacine ou un sel pharmaceutiquement acceptable de celle-ci destinée à être utilisée selon la revendication 1 ou la revendication 2, où l'infection est une urétrite.

5. Doxycycline ou gépotidacine ou un sel pharmaceutiquement acceptable de celle-ci destinée à être utilisée selon la revendication 4, où l'infection est une urétrite non gonococcique.

6. Doxycycline ou gépotidacine ou un sel pharmaceutiquement acceptable de celle-ci destinée à être utilisée selon l'une quelconque des revendications 1 à 5, où le *Mycoplasma genitalium* causant l'infection est résistant à l'azithromycine ou à la moxifloxacine.

7. Doxycycline ou gépotidacine ou un sel pharmaceutiquement acceptable de celle-ci destinée à être utilisée selon l'une quelconque des revendications 1 à 6, où la gépotidacine ou un sel pharmaceutiquement acceptable de celle-ci, et la doxycycline, sont administrés successivement.

8. Gépotidacine ou un sel pharmaceutiquement acceptable de celle-ci destinée à être utilisée selon l'une quelconque des revendications 1 à 7, où la gépotidacine est le méthanesulfonate de gépotidacine.

9. Gépotidacine ou un sel pharmaceutiquement acceptable de celle-ci destinée à être utilisée selon l'une quelconque des revendications 1 à 8, où la gépotidacine ou un sel pharmaceutiquement acceptable de celle-ci est administrée par voie orale.

10. Doxycycline destinée à être utilisée selon l'une quelconque des revendications 1 à 7, où la doxycycline est administrée par voie orale.

11. Kit comprenant de la gépotidacine ou un sel pharmaceutiquement acceptable de celle-ci, et de la doxycycline, destiné à être utilisé dans le traitement d'une infection causée par *Mycoplasma genitalium.*

12. Combinaison pharmaceutique qui comprend de la gépotidacine ou un sel pharmaceutiquement acceptable de celle-ci et de la doxycycline destinée à être utilisée dans le traitement d'une infection causée par *Mycoplasma genitalium.*

13. Composition pharmaceutique qui comprend de la gépotidacine ou un sel pharmaceutiquement acceptable de celle-ci, de la doxycycline et au moins un excipient pharmaceutiquement acceptable destinée à être utilisée dans le traitement d'une infection causée par *Mycoplasma genitalium.*
